# EUROPEAN PATENT SPECIFICATION

(11) **EP 4 529 854 B1**
(45) Date of publication and mention of the grant of the patent: **13.05.2026**
(21) Application number: 24203330.6
(22) Date of filing: 27.09.2024
(51) Int. Cl.: A61B 8/00, A61B 8/08

(54) **ULTRASOUND DIAGNOSTIC APPARATUS AND CONTROL METHOD OF ULTRASOUND DIAGNOSTIC APPARATUS**
ULTRASCHALLDIAGNOSEVORRICHTUNG UND STEUERUNGSVERFAHREN FÜR DIE ULTRASCHALLDIAGNOSEVORRICHTUNG
APPAREIL DE DIAGNOSTIC À ULTRASONS ET PROCÉDÉ DE COMMANDE D'APPAREIL DE DIAGNOSTIC À ULTRASONS

(30) Priority: 28.09.2023 JP 2023167690
(43) Date of publication of application: 02.04.2025
(73) Proprietor: FUJIFILM Corporation, Tokyo 106-8620 (JP)
(72) Inventor: EBATA, Tetsurou, Kanagawa (JP)
(74) Representative: Dehns Germany Partnerschaft mbB

(56) References cited:
- US-A1- 2015 359 517
- US-A1- 2017 281 134
- LIU LI ET AL: "Obstetric Ultrasound Simulator With Task-Based Training and Assessment", IEEE TRANSACTIONS ON BIOMEDICAL ENGINEERING, IEEE, USA, vol. 62, no. 10, 2 October 2015 (2015-10-02), pages 2480 - 2497, XP011668913, ISSN: 0018-9294, [retrieved on 20150916], DOI: 10.1109/TBME.2015.2433679

## Description

### BACKGROUND OF THE INVENTION

### 1. Field of the Invention

The present invention relates to an ultrasound diagnostic apparatus, and a control method of the ultrasound diagnostic apparatus which comprehensively scan an inside of a subject.

### 2. Description of the Related Art

In the related art, an examination is performed by capturing an ultrasound image representing a tomographic image of a subject by using a so-called ultrasound diagnostic apparatus. In such an examination, a user such as a doctor usually captures the ultrasound image while changing a posture of a so-called ultrasound probe and moving the ultrasound probe in a state where the ultrasound probe is in contact with a body surface of the subject.

In this case, the user usually sequentially captures ultrasound images while determining a scanned part of the subject by checking the captured ultrasound image, but a user having a low skill level in the examination using the ultrasound diagnostic apparatus may have difficulty in determining whether or not a site of an examination target in the subject has been comprehensively scanned. Therefore, for example, as disclosed in JP2012-104137A, a technique has been developed in which a contour of an organ as the examination target is extracted from ultrasound images of a plurality of frames captured while the ultrasound probe is moved, and a region in which an interval between the extracted contours is equal to or greater than a certain value is displayed as a region that has not yet been scanned.

US2015/359517 A1 relates to an ultrasound system including a processing unit which is configured to generate a three-dimensional model from ultrasouond images and the position and orientation of the ultrasound probe, using an image reconstruction algorithm.

### SUMMARY OF THE INVENTION

However, in a case of determining whether or not comprehensive scanning has been performed by extracting the contour of the organ as disclosed in JP2012-104137A, for example, even in a case where there is a region that has not yet been scanned in a region inside the contour, it may be determined that the comprehensive scanning is completed. In this case, there is a concern that the user having a low skill level may complete the examination even though the organ has not yet been comprehensively scanned.

The present invention has been made in order to solve such a problem in the related art, and an object thereof is to provide an ultrasound diagnostic apparatus and a control method of the ultrasound diagnostic apparatus according to independent claims 1 and 16. Optional features are defined in the dependent claims.

### BRIEF DESCRIPTION OF THE DRAWINGS

Fig. 1 is a block diagram illustrating a configuration of an ultrasound diagnostic apparatus according to a first embodiment of the present invention.
Fig. 2 is a block diagram illustrating a configuration of a transmission and reception circuit in the first embodiment of the present invention.
Fig. 3 is a block diagram illustrating a configuration of an image generation unit in the first embodiment of the present invention.
Fig. 4 is a schematic view of an ultrasound probe on a body surface of a subject and an organ being scanned.
Fig. 5 is a display example of a contour and a region of an organ in the first embodiment.
Fig. 6 is a flowchart illustrating an operation of the ultrasound diagnostic apparatus according to the first embodiment of the present invention.
Fig. 7 is a block diagram illustrating a configuration of an ultrasound diagnostic apparatus according to a second embodiment of the present invention.
Fig. 8 is a diagram illustrating an example of a three-dimensional grid set in three-dimensional image data of an organ.
Fig. 9 is a diagram in which a three-dimensional grid is partially enlarged.
Fig. 10 is a block diagram illustrating a configuration of an ultrasound diagnostic apparatus according to a third embodiment of the present invention.
Fig. 11 is a block diagram illustrating a configuration of an ultrasound diagnostic apparatus according to a fourth embodiment of the present invention.
Fig. 12 is a block diagram illustrating a configuration of an ultrasound diagnostic apparatus according to a fifth embodiment of the present invention.
Fig. 13 is a diagram illustrating an example of a three-dimensional schema image in the fifth embodiment of the present invention.
Fig. 14 is a block diagram illustrating a configuration of an ultrasound diagnostic apparatus according to a sixth embodiment of the present invention.
Fig. 15 is another display example of a contour and a region of an organ in the sixth embodiment.

### DESCRIPTION OF THE PREFERRED EMBODIMENTS

Hereinafter, embodiments of the present invention will be described with reference to the accompanying drawings.

The description of configuration requirements described below is given on the basis of a representative embodiment of the present invention, but the present invention is not limited to such an embodiment.

Note that, in the present specification, a numerical range represented by using "to" means a range including numerical values before and after "to" as a lower limit value and an upper limit value.

In the present specification, the terms "same" and "identical" include an error range generally allowed in the technical field.

### First Embodiment

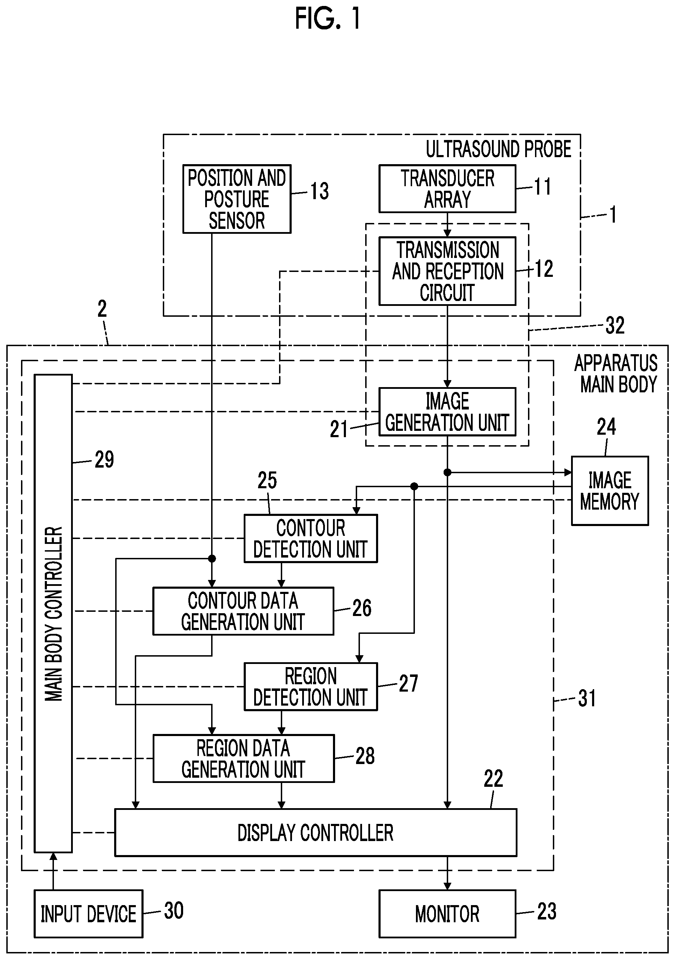

Fig. 1 illustrates a configuration of an ultrasound diagnostic apparatus according to a first embodiment of the present invention. The ultrasound diagnostic apparatus comprises an ultrasound probe 1, and an apparatus main body 2 connected to the ultrasound probe 1. The ultrasound probe 1 and the apparatus main body 2 are connected to each other by so-called wired communication or so-called wireless communication.

The ultrasound probe 1 includes a transducer array 11. A transmission and reception circuit 12 is connected to the transducer array 11. In addition, the ultrasound probe 1 includes a position and posture sensor 13. The position and posture sensor 13 may be built into the ultrasound probe 1, or may be attached to a housing of the ultrasound probe 1. In addition, for example, in a case where a sensor device that measures the ultrasound probe 1 from the outside, such as a so-called optical sensor, is used as the position and posture sensor 13, the position and posture sensor 13 may be disposed outside the ultrasound probe 1.

The apparatus main body 2 includes an image generation unit 21 connected to the transmission and reception circuit 12 of the ultrasound probe 1. A display controller 22 and a monitor 23 are sequentially connected to the image generation unit 21. An image memory 24 is connected to the image generation unit 21. A contour detection unit 25 is connected to the image memory 24. A contour data generation unit 26 is connected to the position and posture sensor 13 and the contour detection unit 25. The contour data generation unit 26 is connected to the display controller 22. In addition, a region detection unit 27 is connected to the image memory 24. A region data generation unit 28 is connected to the position and posture sensor 13 and the region detection unit 27. The region data generation unit 28 is connected to the display controller 22.

In addition, a main body controller 29 is connected to the image generation unit 21, the display controller 22, the image memory 24, the contour detection unit 25, the contour data generation unit 26, the region detection unit 27, and the region data generation unit 28. An input device 30 is connected to the main body controller 29. In addition, the image generation unit 21, the display controller 22, the contour detection unit 25, the contour data generation unit 26, the region detection unit 27, the region data generation unit 28, and the main body controller 29 constitute a processor 31 for the apparatus main body 2.

The transducer array 11 of the ultrasound probe 1 has a plurality of ultrasonic transducers arranged in a one-dimensional or two-dimensional manner. According to a drive signal supplied from the transmission and reception circuit 12, each of the ultrasonic transducers transmits an ultrasonic wave and receives an ultrasound echo from the subject to output a signal based on the ultrasound echo. For example, each ultrasonic transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

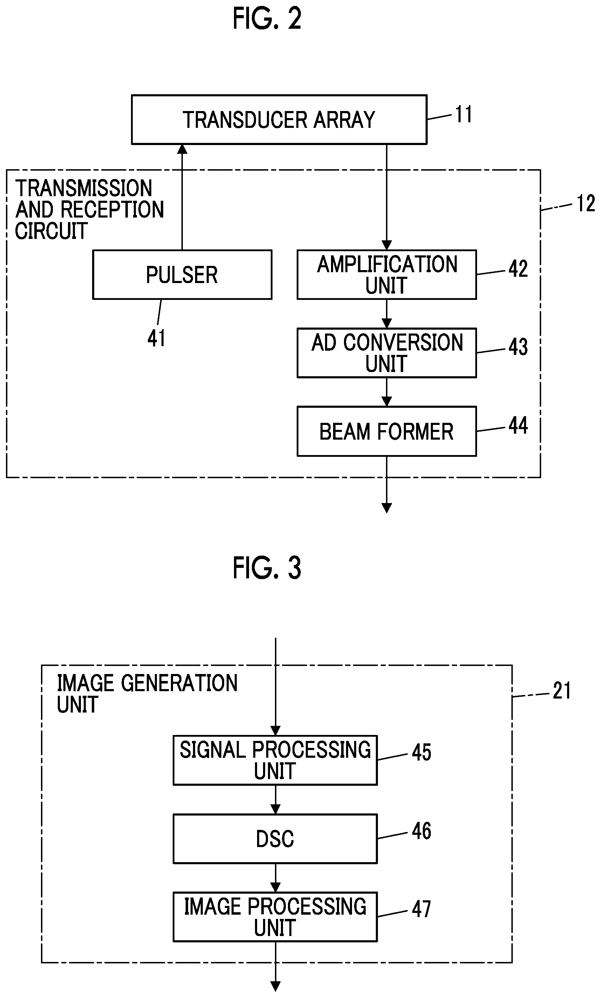

The transmission and reception circuit 12 causes the transducer array 11 to transmit the ultrasonic wave and generates a sound ray signal on the basis of a reception signal acquired by the transducer array 11, under the control of the main body controller 29. As illustrated in Fig. 2, the transmission and reception circuit 12 includes a pulser 41 connected to the transducer array 11, and an amplification unit 42, an analog-to-digital (AD) conversion unit 43, and a beam former 44 that are sequentially connected in series to the transducer array 11.

The pulser 41 includes, for example, a plurality of pulse generators, and the pulser 41 adjusts the amount of delay of each drive signal so that ultrasonic waves transmitted from the plurality of ultrasonic transducers of the transducer array 11 form an ultrasound beam on the basis of a transmission delay pattern selected according to the control signal from the main body controller 29, and supplies the obtained signals to the plurality of ultrasonic transducers. In this way, in a case where a pulsed or continuous wave-like voltage is applied to the electrodes of the ultrasonic transducer of the transducer array 11, the piezoelectric body expands and contracts to generate a pulsed or continuous wave-like ultrasonic wave from each of the ultrasonic transducers, whereby an ultrasound beam is formed from the combined wave of these ultrasonic waves.

The transmitted ultrasound beam is reflected by a target, for example, a site of the subject, and propagates toward the transducer array 11 of the ultrasound probe 1. The ultrasound echo propagating toward the transducer array 11 in this way is received by each of the ultrasonic transducers constituting the transducer array 11. In this case, each of the ultrasonic transducers constituting the transducer array 11 receives the propagating ultrasound echo to expand and contract to generate a reception signal, which is an electrical signal, and outputs these reception signals to the amplification unit 42.

The amplification unit 42 amplifies the signal input from each of the ultrasonic transducers constituting the transducer array 11, and transmits the amplified signal to the AD conversion unit 43. The AD conversion unit 43 converts the signal transmitted from the amplification unit 42 into digital reception data. The beam former 44 performs so-called reception focus processing by applying and adding a delay to each reception data received from the AD conversion unit 43. With the reception focus processing, each piece of reception data converted in the AD conversion unit 43 is subjected to phasing addition, and a sound ray signal in which a focus of the ultrasound echo is narrowed is acquired.

As illustrated in Fig. 3, the image generation unit 21 has a configuration in which a signal processing unit 45, a digital scan converter (DSC) 46, and an image processing unit 47 are sequentially connected in series.

The signal processing unit 45 generates a B-mode image signal, which is tomographic image information regarding tissues inside the subject, by performing, on the sound ray signal received from the transmission and reception circuit 12, correction of the attenuation due to the distance according to the depth of the reflection position of the ultrasonic wave by using a sound velocity value set by the main body controller 29 and then performing envelope detection processing.

The DSC 46 converts (raster-converts) the B-mode image signal generated by the signal processing unit 45 into an image signal in accordance with a normal television signal scanning method.

The image processing unit 47 performs various kinds of necessary image processing such as gradation processing on the B-mode image signal input from the DSC 46, and then transmits the B-mode image signal to the display controller 22 and the image memory 24. Hereinafter, the B-mode image signal that has been subjected to image processing by the image processing unit 47 is referred to as an ultrasound image.

The image memory 24 is a memory that stores the ultrasound image acquired by an image acquisition unit 32. Here, as the image memory 24, for example, recording media such as a flash memory, a hard disk drive (HDD), a solid state drive (SSD), a flexible disk (FD), a magneto-optical disk (MO disk), a magnetic tape (MT), a random access memory (RAM), a compact disc (CD), a digital versatile disc (DVD), a secure digital card (SD card), or a universal serial bus memory (USB memory) can be used.

The position and posture sensor 13 of the ultrasound probe 1 is a sensor device that acquires position and posture information of the ultrasound probe 1. Here, in general, in a case where a user performs an examination of a subject by using an ultrasound diagnostic apparatus, the user often performs the examination while changing a posture, that is, an angle of the ultrasound probe 1 and moving the position of the ultrasound probe 1 in a state where the ultrasound probe 1 is in contact with the body surface of the subject. The position and posture information of the ultrasound probe 1 acquired by the position and posture sensor 13 includes information regarding the posture and position of the ultrasound probe 1. For example, the position and posture sensor 13 can include at least one of a so-called inertial sensor, a magnetic sensor, or an optical sensor. The inertial sensor can include, for example, at least one of a so-called acceleration sensor or a gyro sensor.

The contour detection unit 25 detects a contour of an organ being imaged in an ultrasound image on the basis of the ultrasound image acquired by the image acquisition unit 32. The contour detection unit 25 can detect the contour of the organ by using, for example, a contour detection model in which the contour of the organ in the ultrasound image in which the organ is imaged is trained in advance.

Here, the contour detection model is a so-called machine learning model, and a so-called deep learning model, a so-called support vector machine (SVM), a so-called decision tree model, or the like can be used as the contour detection model. The contour detection model learns a relationship between a large number of ultrasound images in which an organ is imaged and a contour of the organ in the ultrasound images in advance, and outputs the contour of the organ in the ultrasound image in response to the input of the ultrasound image in which the organ is imaged.

In addition to a method using the contour detection model, the contour detection unit 25 can also detect the contour of the organ by using, for example, a method of so-called scale-invariant feature transform (SIFT), a method of edge detection based on a change in brightness in the ultrasound image, or the like.

The contour data generation unit 26 generates three-dimensional image data of the contour of the organ on the basis of the contour detected by the contour detection unit 25 and the position and posture information acquired by the position and posture sensor 13. In this case, for example, the contour data generation unit 26 can generate the three-dimensional image data of the contour of the organ by associating the contour of the organ detected by the contour detection unit 25 with the position and posture information of the ultrasound probe 1 acquired by the position and posture sensor 13 at the timing at which the ultrasound image in which the contour of the organ is detected is acquired and sequentially arranging the contour of the organ in a three-dimensional space in accordance with the corresponding position and posture information.

The contour data generation unit 26 can associate, for example, the position and posture information acquired by the position and posture sensor 13 with the contour on which the detection processing has been performed by the contour detection unit 25 at the time at which the position and posture information is acquired. In addition, in a case where a time stamp indicating an acquisition time point is added to the position and posture information in the position and posture sensor 13 and a time stamp indicating the acquisition time point is added to the ultrasound image in the image acquisition unit 32, for example, the contour data generation unit 26 can associate the position and posture information with the contour of the organ detected from the ultrasound image in a case where a difference between the time stamp added to the position and posture information and the time stamp added to the ultrasound image is within a certain range. For example, in a case where a generation rate of the ultrasound image by the image acquisition unit 32 is slower than an acquisition rate of the position and posture information by the position and posture sensor 13, the contour data generation unit 26 can associate the position and posture information having a time stamp indicating a time point close to the time stamp of the ultrasound image with the contour detected from the ultrasound image, on the basis of the time stamp of the ultrasound image.

The region detection unit 27 detects a region of an organ being imaged in the ultrasound image on the basis of the ultrasound image acquired by the image acquisition unit 32. Here, the region of the organ refers to a part including tissues of the organ. The region detection unit 27 can detect the region of the organ by using, for example, a region detection model in which the region of the organ in the ultrasound image in which the organ is imaged is trained.

Here, the region detection model is a machine learning model that performs so-called segmentation processing on the ultrasound image, and a deep learning model, a support vector machine (SVM), a decision tree model, or the like can be used as the region detection model. The region detection model learns a relationship between a large number of ultrasound images in which an organ is imaged and a region of the organ in the ultrasound images in advance, and outputs the region of the organ in the ultrasound image for each pixel in response to the input of the ultrasound image in which the organ is imaged.

The region data generation unit 28 generates three-dimensional image data of the region of the organ on the basis of the region of the organ detected by the region detection unit 27 and the position and posture information of the ultrasound probe 1 acquired by the position and posture sensor 13. In this case, for example, the region data generation unit 28 can generate the three-dimensional image data of the region of the organ by associating the region of the organ detected by the region detection unit 27 with the position and posture information of the ultrasound probe 1 acquired by the position and posture sensor 13 at the timing at which the ultrasound image in which the region of the organ is detected is acquired and sequentially arranging the region of the organ in a three-dimensional space in accordance with the corresponding position and posture information.

The region data generation unit 28 can associate, for example, the position and posture information input from the position and posture sensor 13 with the region of the organ input at a time point at which a period of time required for the region detection unit 27 to detect the region has elapsed from the input of the position and posture information. In addition, in a case where a time stamp indicating an acquisition time point is added to the position and posture information in the position and posture sensor 13 and a time stamp indicating the acquisition time point is added to the ultrasound image in the image acquisition unit 32, for example, the region data generation unit 28 can associate the position and posture information with the region of the organ detected from the ultrasound image in a case where a difference between the time stamp added to the position and posture information and the time stamp added to the ultrasound image is within a certain range.

The display controller 22 performs predetermined processing on the ultrasound image transmitted from the image acquisition unit 32, an image based on the three-dimensional image data of the contour of the organ generated by the contour data generation unit 26, and an image based on the three-dimensional image data of the region of the organ generated by the region data generation unit 28 to display the resultant on the monitor 23, under the control of the main body controller 29.

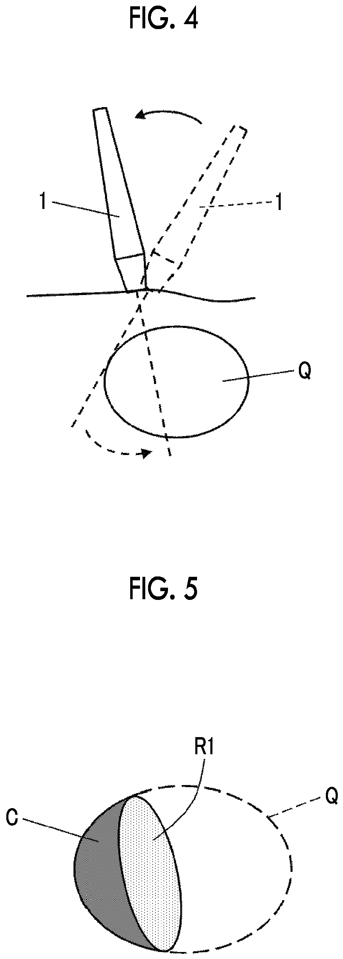

Here, for example, as illustrated in Fig. 4, in a case where ultrasound images of an organ Q in the subject are sequentially captured while the posture of the ultrasound probe 1 is changed, the ultrasound images of the plurality of frames corresponding to the different postures of the ultrasound probe 1 are acquired, the contour data generation unit 26 sequentially generates the three-dimensional image data of the contour of the organ Q in a scanning range of the ultrasound probe 1, and the region data generation unit 28 sequentially generates the three-dimensional image data of the region of the organ Q in the scanning range of the ultrasound probe 1.

In this case, for example, as illustrated in Fig. 5, the display controller 22 can cumulatively display, on the basis of three-dimensional image data of a contour C of the organ Q and three-dimensional image data of a region R1 of the organ Q that are sequentially generated, an image representing a three-dimensional shape of the contour C and an image representing a three-dimensional shape of the region R1 on the monitor 23. For example, the display controller 22 can assign colors with different transparency to the image representing the three-dimensional shape of the contour C and the image representing the three-dimensional shape of the region R1 such that the user can check both the image representing the three-dimensional shape of the contour C and the image representing the three-dimensional shape of the region R1. Here, in the example of Fig. 5, the organ Q is illustrated as an ellipsoid for simplification of the description.

In a state where only a part of the organ Q is scanned, an image representing the contour C of the part of the organ Q and the region R1 of the part of the organ Q is displayed on the monitor 23. However, in a case where the organ Q is scanned comprehensively, an image representing the contour C covering the entire surface of the organ Q and an image representing the region R1 filling all the parts surrounded by the surface of the organ Q are displayed on the monitor 23. Therefore, the user can easily ascertain a progress status of the scanning by performing the scanning with the ultrasound probe 1 while checking the image representing the three-dimensional shape of the contour C and the image representing the three-dimensional shape of the region R1 displayed on the monitor 23, and can reliably perform the comprehensive scanning of the organ Q.

Note that the display controller 22 can also display the image representing the three-dimensional shape of the contour C and the image representing the three-dimensional shape of the region R1 adjacent to each other on the monitor 23 instead of displaying the image representing the three-dimensional shape of the contour C and the image representing the three-dimensional shape of the region R1 in a superimposed manner on the monitor 23.

The monitor 23 is for displaying the ultrasound image, the image representing the contour C and the region R1 of the organ Q, and the like under the control of the display controller 22, and includes a display device such as a liquid crystal display (LCD), or an organic electroluminescence (EL) display.

The main body controller 29 controls each unit of the apparatus main body 2 and the transmission and reception circuit 12 of the ultrasound probe 1 on the basis of a control program and the like stored in advance.

The input device 30 is for a user to perform an input operation, and is configured by, for example, a device such as a keyboard, a mouse, a trackball, a touchpad, and a touch sensor superimposed on the monitor 23.

Note that the processor 31 having the image generation unit 21, the display controller 22, the contour detection unit 25, the contour data generation unit 26, the region detection unit 27, the region data generation unit 28, and the main body controller 29 is configured by a central processing unit (CPU) and a control program for causing the CPU to execute various kinds of processing, but the processor 31 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 21, the display controller 22, the contour detection unit 25, the contour data generation unit 26, the region detection unit 27, the region data generation unit 28, and the main body controller 29 of the processor 31 can be partially or wholly integrated into one CPU or the like.

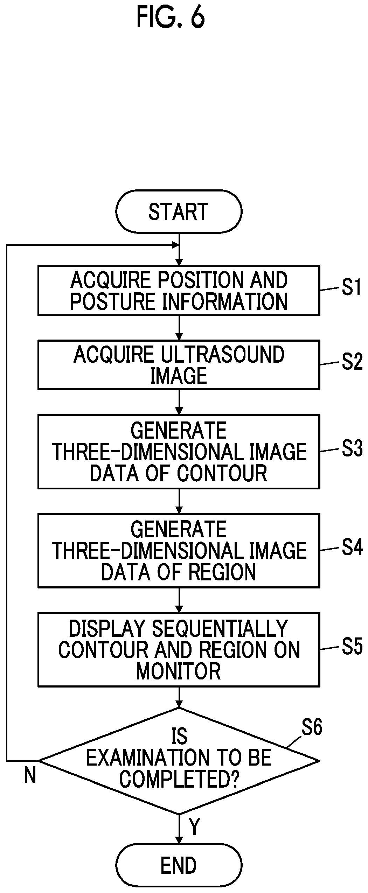

Next, the operation of the ultrasound diagnostic apparatus according to the first embodiment will be described with reference to the flowchart illustrated in Fig. 6. In the following description, it is assumed that the user performs the scanning with the ultrasound probe 1 while changing the position and the posture of the ultrasound probe 1 in a state where the ultrasound probe 1 is in contact with the body surface of the subject in order to scan the organ Q comprehensively.

In step S1, the position and posture sensor 13 acquires the position and posture information of the ultrasound probe 1. The position and posture information of the ultrasound probe 1 acquired in step S1 is transmitted to the contour data generation unit 26 and the region data generation unit 28.

In step S2, the image acquisition unit 32 acquires the ultrasound image of the subject. In this case, under the control of the main body controller 29, the transmission and reception of ultrasonic waves from the plurality of transducers of the transducer array 11 are started according to the drive signal from the pulser 41 of the transmission and reception circuit 12 of the ultrasound probe 1, the ultrasound echo from the subject is received by the plurality of transducers of the transducer array 11, and the reception signal as the analog signal is output to the amplification unit 42 to be amplified, and then is subjected to the AD conversion by the AD conversion unit 43 to acquire the reception data.

The reception focus processing is performed on the reception data by the beam former 44, and the sound ray signal generated by the reception focusing processing is transmitted to the image generation unit 21 of the apparatus main body 2. An ultrasound image representing tomographic image information of the subject is generated by the image generation unit 21. In this case, the signal processing unit 45 of the image generation unit 21 performs the correction of the attenuation according to the depth of the reflection position of the ultrasonic wave and the envelope detection processing on the sound ray signal, the DSC 46 performs the conversion into the image signal according to a normal television signal scanning method, and the image processing unit 47 performs various kinds of necessary image processing such as gradation processing. The ultrasound image acquired in step S2 in this way is displayed on the monitor 23 via the display controller 22, and is transmitted to the contour detection unit 25 and the region detection unit 27 via the image memory 24.

In step S3, the contour detection unit 25 detects the contour C of the organ Q imaged in the ultrasound image acquired in step S2, and the contour data generation unit 26 generates the three-dimensional image data of the contour C of the organ Q on the basis of the contour C of the organ Q detected by the contour detection unit 25 and the position and posture information of the ultrasound probe 1 acquired in step S1.

The contour detection unit 25 can detect the contour C of the organ Q imaged in the ultrasound image by, for example, a method of using a contour detection model in which the contour C of the organ Q in the ultrasound image in which the organ Q is imaged is trained in advance.

In addition, the contour data generation unit 26 generates the three-dimensional image data of the contour C of the organ Q by associating the contour C of the organ Q detected by the contour detection unit 25 with the position and posture information of the ultrasound probe 1 and disposing the contour C of the organ Q in the three-dimensional space on the basis of the position and posture information of the ultrasound probe 1.

The contour data generation unit 26 can associate, for example, the position and posture information input from the position and posture sensor 13 with the contour C of the organ Q input at a time point at which a period of time required for the contour detection unit 25 to detect the contour C has elapsed from the input of the position and posture information.

In step S4, the region detection unit 27 detects the region R1 of the organ Q imaged in the ultrasound image acquired in step S2, and the region data generation unit 28 generates the three-dimensional image data of the region R1 of the organ Q on the basis of the region R1 of the organ Q detected by the region detection unit 27 and the position and posture information of the ultrasound probe 1 acquired in step S1.

The region detection unit 27 can detect the region R1 of the organ Q imaged in the ultrasound image by, for example, a method of using a region detection model in which the region R1 of the organ Q in the ultrasound image in which the organ Q is imaged is trained in advance.

In addition, the region data generation unit 28 generates the three-dimensional image data of the region R1 of the organ Q by associating the region R1 of the organ Q detected by the region detection unit 27 with the position and posture information of the ultrasound probe 1 and disposing the region R1 of the organ Q in the three-dimensional space on the basis of the position and posture information of the ultrasound probe 1.

In step S5, the display controller 22 displays the image representing the contour C of the organ Q and the image representing the region R1 of the organ Q on the monitor 23 on the basis of the three-dimensional image data of the contour C of the organ Q generated in step S3 and the three-dimensional image data of the region R1 of the organ Q generated in step S4. In this case, for example, as illustrated in Fig. 5, the display controller 22 can display the image representing the three-dimensional shape of the contour C and the region R1 inside the contour C of the organ Q on the monitor 23.

In step S6, the main body controller 29 determines whether or not to complete the examination of the subject using the ultrasound diagnostic apparatus. The main body controller 29 determines, for example, to complete the examination of the subject in a case where the user determines that the organ Q has been sufficiently scanned and inputs an instruction to complete the examination via the input device 30, and determines to continue the examination in a case where the user does not particularly input an instruction to complete the examination via the input device 30.

In a case where it is determined to continue the examination in step S6, the processing returns to step S1, and the position and posture information of the ultrasound probe 1 is acquired by the position and posture sensor 13. Since the user performs the scanning with the ultrasound probe 1 while changing the position and the posture of the ultrasound probe 1, the position and posture information acquired in current step S1 represents a position and a posture different from the position and the posture of the ultrasound probe 1 represented by the position and posture information acquired in previous step S1.

Next, in step S2, a new ultrasound image is acquired.

In subsequent step S3, three-dimensional image data of the contour C of the organ Q imaged in the ultrasound image acquired in step S2 is generated. In this case, the contour data generation unit 26 generates the three-dimensional image data of the contour C by arranging the contour C of the organ Q detected by the contour detection unit 25 in previous step S3 and the contour of the organ Q detected by the contour detection unit 25 in current step S3 in the three-dimensional space in accordance with the corresponding position and posture information.

In step S4, the three-dimensional image data of the region R1 of the organ Q imaged in the ultrasound image acquired in step S2 is generated. In this case, the region data generation unit 28 generates the three-dimensional image data of the region R1 by arranging the region R1 of the organ Q detected by the region detection unit 27 in previous step S4 and the region R1 of the organ Q detected by the region detection unit 27 in current step S4 in the three-dimensional space in accordance with the corresponding position and posture information.

In step S5, the display controller 22 displays the image representing the contour C and the region R1 of the organ Q on the monitor 23 on the basis of the three-dimensional image data of the contour C and the region R1 of the organ Q generated in previous steps S3 and S4.

In a case where the processing of step S5 is completed, the main body controller 29 determines in step S6 whether or not to complete the examination of the subject.

In this manner, the processing of steps S1 to S6 is repeated as long as it is determined in step S6 to continue the examination of the subject. Accordingly, the contour C of the organ Q displayed on the monitor 23 is gradually enlarged along the surface of the organ Q, and the region R1 of the organ Q gradually fills the inside of the organ Q. Finally, in a case where the organ Q is scanned comprehensively, the image representing the contour C covering the entire surface of the organ Q and the image representing the region R1 filling all the parts surrounded by the surface of the organ Q are displayed on the monitor 23.

The user can easily ascertain the progress status of the scanning and can reliably perform the comprehensive scanning of the organ Q by performing the scanning with the ultrasound probe 1 while checking the image representing the contour C and the region R1 of the organ Q displayed on the monitor 23 in repetition of steps S1 to S6.

In a case where it is determined in step S6 to complete the examination of the subject, the operation of the ultrasound diagnostic apparatus according to the flowchart of Fig. 6 is completed.

As described above, with the ultrasound diagnostic apparatus according to the first embodiment, the contour detection unit 25 detects the contour C of the organ Q imaged in the ultrasound image, the region detection unit 27 detects the region R1 of the organ Q imaged in the ultrasound image, the contour data generation unit 26 generates the three-dimensional image data of the contour C of the organ Q on the basis of the contour C of the organ Q and the position and posture information, the region data generation unit 28 generates the three-dimensional image data of the region R1 of the organ Q on the basis of the region R1 of the organ Q and the position and posture information, and the display controller 22 sequentially displays the image representing the contour C and the image representing the region R1 on the monitor 23 on the basis of the three-dimensional image data of the contour C and the three-dimensional image data of the region R1. Therefore, the user can reliably perform the comprehensive scanning of the organ Q.

Note that the description has been made in which the transmission and reception circuit 12 is included in the ultrasound probe 1, but the transmission and reception circuit 12 may be included in the apparatus main body 2.

In addition, the description has been made in which the image generation unit 21 is included in the apparatus main body 2, but the image generation unit 21 may be included in the ultrasound probe 1.

In addition, the apparatus main body 2 may be a so-called stationary type, a portable type that is easy to carry, or a so-called handheld type configured by a smartphone or tablet computer. As described above, the type of equipment constituting the apparatus main body 2 is not particularly limited.

In addition, the description has been made in which the display controller 22 displays the image representing the three-dimensional shape of the contour C and the region R1 of the organ Q on the monitor 23, but, for example, in this case, the display controller 22 can display the image representing the three-dimensional shape on the monitor 23 while changing a rotation angle of the three-dimensional shape displayed on the monitor 23 by a user's input operation via the input device 30. In a case where a part of the contour C or a part of the region R1 cannot be detected due to some reason such as that the ultrasound image is unclear, a part of the image representing the contour C of the organ Q or a part of the image representing the region R1 is missing in a case of being displayed. However, even in this case, the user can clearly ascertain a portion that has been scanned and a portion that has not been scanned in the organ Q.

In addition, step S2 is performed after step S1 in the flowchart of Fig. 6, but step S1 may be performed after step S2, or step S1 and step S2 may be simultaneously performed.

In addition, step S4 is performed after step S3, but step S3 may be performed after step S4, or step S3 and step S4 may be simultaneously performed.

### Second Embodiment

In order to enable the user to more reliably perform the comprehensive scanning of the organ Q, the ultrasound diagnostic apparatus determines a portion of which the contour C is not detected or a portion of which the region R1 is not detected in the organ Q, as a non-depicted portion, and can notify the user that the non-depicted portion is present.

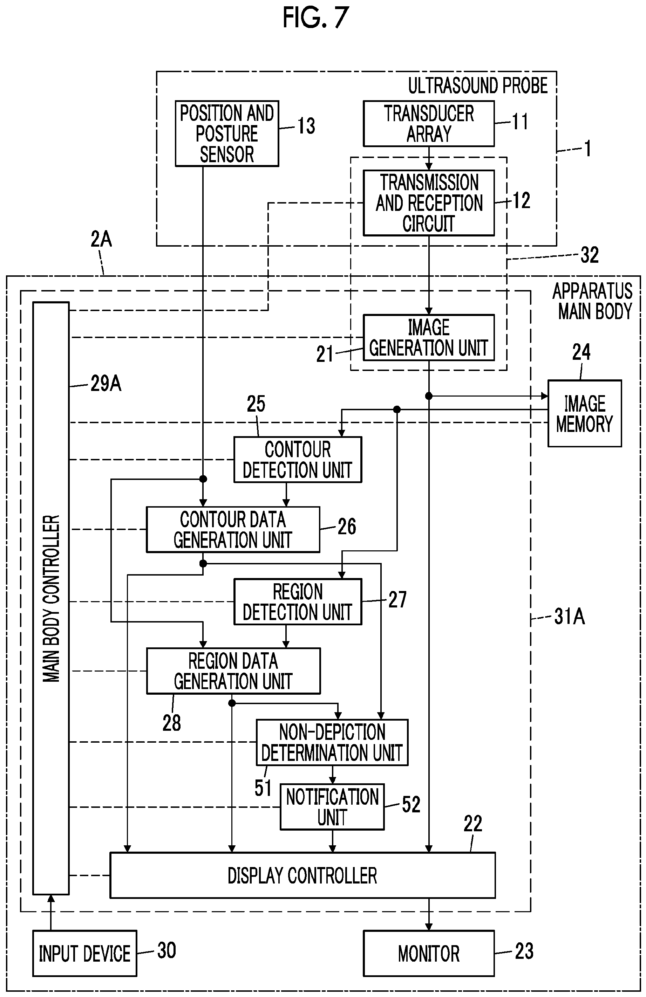

Fig. 7 illustrates a block diagram illustrating a configuration of an ultrasound diagnostic apparatus according to a second embodiment. The ultrasound diagnostic apparatus of the second embodiment is obtained by including an apparatus main body 2Ainstead of the apparatus main body 2 in the ultrasound diagnostic apparatus of the first embodiment illustrated in Fig. 1. The apparatus main body 2A is obtained by further providing a non-depiction determination unit 51 and a notification unit 52 to the apparatus main body 2 in the first embodiment, and including a main body controller 29A instead of the main body controller 29.

In the apparatus main body 2A, the non-depiction determination unit 51 is connected to the contour data generation unit 26 and the region data generation unit 28. The notification unit 52 is connected to the non-depiction determination unit 51. The notification unit 52 is connected to the display controller 22. In addition, the non-depiction determination unit 51 and the notification unit 52 are connected to the main body controller 29A. In addition, the image generation unit 21, the display controller 22, the contour detection unit 25, the contour data generation unit 26, the region detection unit 27, the region data generation unit 28, the main body controller 29A, the non-depiction determination unit 51, and the notification unit 52 constitute a processor 31A for the apparatus main body 2A.

The non-depiction determination unit 51 determines, as the non-depicted portion, a portion of which the contour C of the organ Q is not detected by the contour detection unit 25 or a portion of which the region R1 of the organ Q is not detected by the region detection unit 27 due to some reason unintended by the user, such as a case where the user fails to operate the ultrasound probe 1 or a case where the contour detection unit 25 fails to detect the contour C or the region detection unit 27 fails to detect the region R1 due to the ultrasound image being unclear.

For example, in a case where the contour C of the organ Q detected by the contour detection unit 25 or the region R1 of the organ Q detected by the region detection unit 27 has a discontinuous portion having an interval equal to or greater than a predetermined interval due to some failure, the non-depiction determination unit 51 can determine the discontinuous portion as the non-depicted portion.

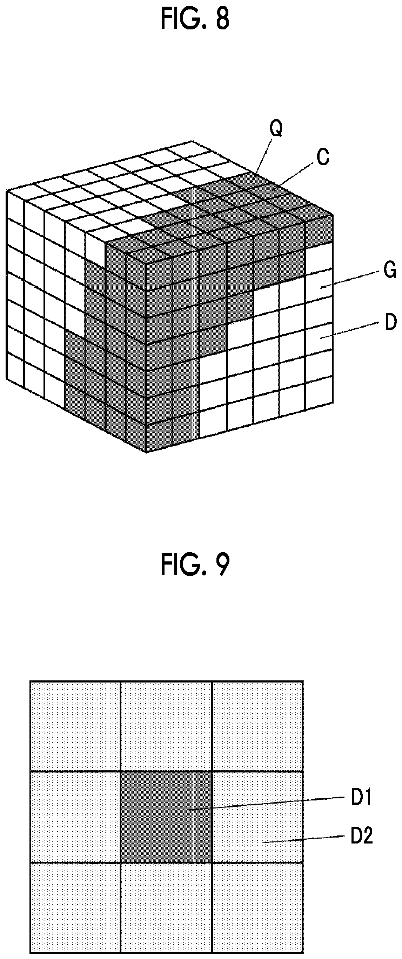

In this case, for example, as illustrated in Fig. 8, the non-depiction determination unit 51 can set a three-dimensional grid G consisting of a plurality of cells D having a certain size of a cubic shape with respect to the three-dimensional image data of the contour C and the region R1 of the organ Q. As illustrated in Fig. 9, in a case where any one of four adjacent cells D2 in contact with the four sides of a cell D1 occupied by the contour C or the region R1 in the three-dimensional grid G is not occupied by the contour C or the region R1, the non-depiction determination unit 51 can regard the adjacent cells D2 as the discontinuous portion and determine the adjacent cells D2 as the non-depicted portion. In addition, in a case where any one of eight adjacent cells D2 positioned around the cell D1 occupied by the contour C or the region R1 is not occupied by the contour C or the region R1, the non-depiction determination unit 51 can regard the adjacent cells D2 as the discontinuous portion and determine the adjacent cells D2 as the non-depicted portion.

In addition, in a case where a predetermined period of time has elapsed from the start of the scanning by the ultrasound probe 1, the non-depiction determination unit 51 can determine that the non-depicted portion is present. In a state where a sufficient period of time has elapsed from the start of the scanning, the user has likely already scanned most of the organ Q, but the user can find an omission in scanning by the non-depiction determination unit 51 determining that the non-depicted portion is present.

In addition, the non-depiction determination unit 51 can also determine that the non-depicted portion is present in a case where the user gives an instruction to complete the examination. Even in a case where it is recognized that the user has sufficiently scanned the organ Q, there may be a location that has not yet been scanned, and therefore, it is possible to find an omission in scanning by the non-depiction determination unit 51 determining that the non-depicted portion is present.

In addition, the non-depiction determination unit 51 can determine whether or not the contour C detected by the contour detection unit 25 is closed, and in a case where the contour C is closed, the non-depiction determination unit 51 can determine that the non-depicted portion of which the region R1 is not detected by the region detection unit 27 is present. Even in a case where all the contours C of the organ Q are detected, there may be a case in which a portion surrounded by the contour C cannot be detected due to some reason such as the presence of an unclear portion in the ultrasound image. Therefore, it is possible to find an omission in scanning by the non-depiction determination unit 51 determining that the non-depicted portion is present.

Here, for example, the non-depiction determination unit 51 stores an organ shape model representing a general shape of each organ Q in advance, and can determine whether or not the contour C is closed by comparing the contour C sequentially detected by the contour detection unit 25 with the organ shape model corresponding to the organ Q of which the contour C is detected. In this case, the non-depiction determination unit 51 can exclude, for example, the portion that is not usually depicted in the ultrasound image, such as a vicinity of a portal vein in the liver, from each organ shape model and store the portion.

Note that, in general, since the organ Q is connected to another adjacent organ Q, it may be difficult to accurately determine a boundary between the organ Q and the other organ Q. Therefore, for example, in a case where the volume of the portion of which the region R1 is detected is equal to or greater than a certain value in the volume surrounded by all the contours C of the organ Q, the non-depiction determination unit 51 can determine that the organ Q is comprehensively scanned and the non-depicted portion is not present. In this case, the non-depiction determination unit 51 can store a volume threshold value related to the volume of the portion of which the region R1 is detected, for example, for each type of organ Q, such as 95% in a case of the bladder, 90% in a case of the liver and the kidney, and 95% in a case of the heart.

In a case where the non-depiction determination unit 51 determines that the non-depicted portion is present, the notification unit 52 notifies the user of the presence of the non-depicted portion, for example, by displaying that the non-depicted portion is present on the monitor 23. With this notification, the user can reliably perform the comprehensive scanning of the organ Q by moving the ultrasound probe 1 to scan the non-depicted portion.

Note that, in a case where the contour C of the organ Q detected by the contour detection unit 25 is closed and the region R1 that is not detected by the region detection unit 27 is present in a range inside the contour C, the notification unit 52 can notify the user that the scanning is completed. The user can further reliably perform the comprehensive scanning of the organ Q by continuing the scanning of the ultrasound probe 1 until this notification is performed.

In addition, as illustrated in Fig. 9, the non-depiction determination unit 51 determines the non-depicted portion with reference to four adjacent cells D2 two-dimensionally adjacent to the cell D1 occupied by the contour C or the region R1 or eight adjacent cells D2 positioned around the cell D1 occupied by the contour C or the region R1, but the non-depiction determination unit 51 can also determine the non-depicted portion with reference to six adjacent cells D2 three-dimensionally in contact with six surfaces of the cell D1 occupied by the contour C or the region R1 or 26 adjacent cells D2 positioned around the cell D1 in the three-dimensional space.

### Third Embodiment

In general, the user often separates the ultrasound probe 1 from the body surface of the subject in a case where the imaging of the subject is ended. Therefore, the ultrasound diagnostic apparatus can determine whether or not the non-depicted portion is present at a timing at which the ultrasound probe 1 is separated from the body surface of the subject.

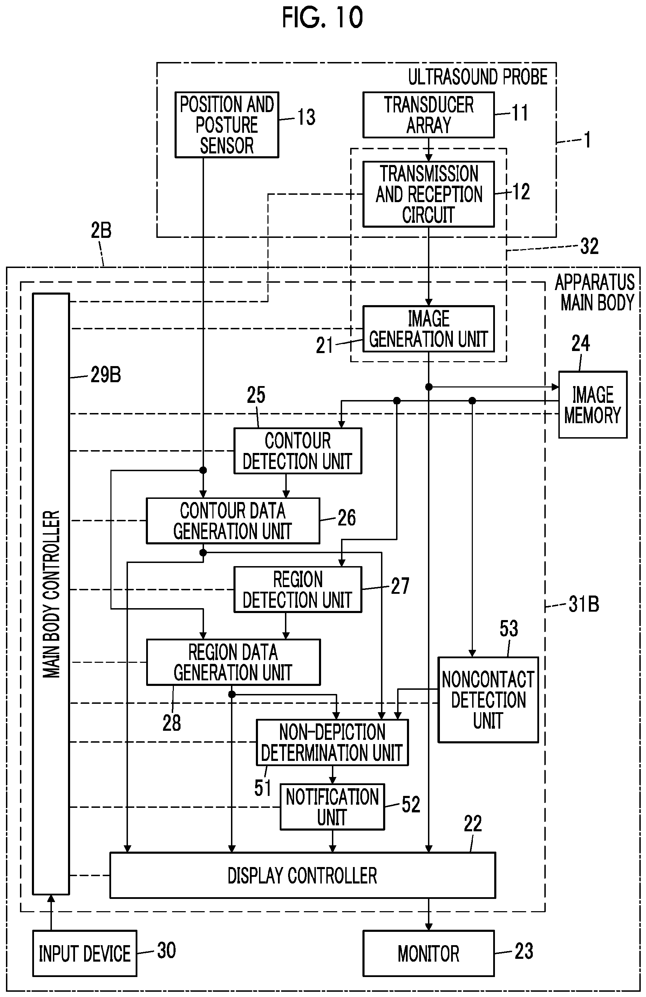

Fig. 10 is a block diagram illustrating a configuration of an ultrasound diagnostic apparatus according to a third embodiment. The ultrasound diagnostic apparatus of the third embodiment is obtained by including an apparatus main body 2B instead of the apparatus main body 2A in the ultrasound diagnostic apparatus of the second embodiment illustrated in Fig. 7. The apparatus main body 2B is obtained by further providing a noncontact detection unit 53 to the apparatus main body 2A in the second embodiment, and including a main body controller 29B instead of the main body controller 29A.

In the apparatus main body 2B, the noncontact detection unit 53 is connected to the image memory 24. The noncontact detection unit 53 is connected to the non-depiction determination unit 51 and the main body controller 29B. In addition, the image generation unit 21, the display controller 22, the contour detection unit 25, the contour data generation unit 26, the region detection unit 27, the region data generation unit 28, the main body controller 29B, the non-depiction determination unit 51, the notification unit 52, and the noncontact detection unit 53 constitute a processor 31B for the apparatus main body 2B.

The noncontact detection unit 53 detects a noncontact state in which the ultrasound probe 1 is separated from the body surface of the subject. Here, in a case where the ultrasound probe 1 is separated from the body surface of the subject, the ultrasonic waves are emitted into the air from the ultrasound probe 1. In this case, an ultrasound echo cannot be obtained, and thus, an aerial radiation image in which the entire image is filled with one color such as black is acquired as the ultrasound image. Therefore, the noncontact detection unit 53 can detect that the ultrasound probe 1 is in the noncontact state by, for example, analyzing the ultrasound image to detect the aerial radiation image.

In a case where the noncontact state of the ultrasound probe 1 is detected by the noncontact detection unit 53, the non-depiction determination unit 51 determines that the non-depicted portion is present.

In a case where the non-depiction determination unit 51 determines that the non-depicted portion is present, the notification unit 52 notifies the user of the determination result.

In general, the user often separates the ultrasound probe 1 from the body surface of the subject in a case where the imaging of the subject is ended. Therefore, by determining that the non-depicted portion is present at a timing at which the noncontact state of the ultrasound probe 1 is detected, the user can find an omission of the scanning and can reliably perform the comprehensive scanning of the organ Q.

Note that the description has been made in which the noncontact detection unit 53 detects the noncontact state of the ultrasound probe 1 by analyzing the ultrasound image to detect the aerial radiation image, but a method of detecting the noncontact state is not particularly limited thereto. For example, in a case where a distal end of the ultrasound probe 1 is provided with a pressure sensor (not illustrated) that measures the pressure of the ultrasound probe 1 coming into contact with the body surface of the subject, the noncontact detection unit 53 can detect the noncontact state of the ultrasound probe 1 on the basis of a measurement value of the pressure sensor. In this case, the noncontact detection unit 53 has, for example, a predetermined pressure threshold value with respect to the measurement value of the pressure sensor, and can detect the noncontact state by determining that the ultrasound probe 1 is not in contact with the subject in a case where the measurement value of the pressure sensor is equal to or less than the pressure threshold value.

### Fourth Embodiment

In order to prevent the omission of the scanning, the ultrasound diagnostic apparatus may notify the user to move the ultrasound probe 1 such that the contour C is depicted in a case where the contour C that can be depicted in the ultrasound image of the organ Q is not detected.

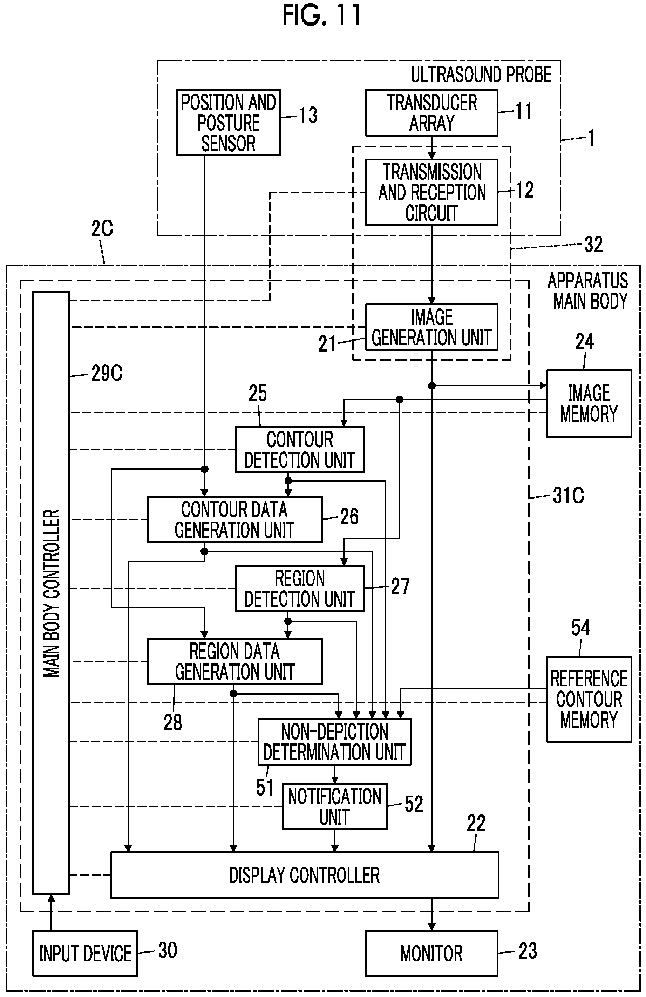

Fig. 11 illustrates a block diagram illustrating a configuration of an ultrasound diagnostic apparatus according to a fourth embodiment. The ultrasound diagnostic apparatus of the fourth embodiment is obtained by including an apparatus main body 2C instead of the apparatus main body 2A in the ultrasound diagnostic apparatus of the second embodiment illustrated in Fig. 7. The apparatus main body 2C in the fourth embodiment is obtained by further providing a reference contour memory 54 to the apparatus main body 2A in the second embodiment, and including a main body controller 29C instead of the main body controller 29A.

In the apparatus main body 2C, the reference contour memory 54, the contour detection unit 25, the contour data generation unit 26, the region detection unit 27, and the region data generation unit 28 are connected to the non-depiction determination unit 51 and the main body controller 29C. In addition, the image generation unit 21, the display controller 22, the contour detection unit 25, the contour data generation unit 26, the region detection unit 27, the region data generation unit 28, the main body controller 29C, the non-depiction determination unit 51, and the notification unit 52 constitute a processor 31C for the apparatus main body 2C.

The reference contour memory 54 is a memory in which a plurality of reference contours that can be depicted in the plurality of organs Q are stored. As the reference contour memory 54, for example, recording media such as a flash memory, an HDD, an SSD, an FD, an MO disk, an MT, a RAM, a CD, a DVD, an SD card, or a USB memory can be used.

The non-depiction determination unit 51 recognizes the organ Q on the basis of the contour C of the organ Q detected by the contour detection unit 25, the three-dimensional image data of the contour C generated by the contour data generation unit 26, the region R1 of the organ Q detected by the region detection unit 27, or the three-dimensional image data of the region R1 generated by the region data generation unit 28, and reads out the plurality of reference contours related to the recognized organ Q from the reference contour memory 54. The non-depiction determination unit 51 determines whether or not the contour C corresponding to the reference contour is detected by comparing the plurality of reference contours read out from the reference contour memory 54 with the plurality of contours C of the organ Q detected by the contour detection unit 25.

The non-depiction determination unit 51 can recognize the organ Q and determine whether or not the contour C corresponding to the reference contour is detected by using, for example, an image analysis technique using a feature amount such as a so-called template matching, AdaBoost, an SVM, or an SIFT, or a machine learning model trained by using a machine learning technique such as deep learning.

In a case where the non-depiction determination unit 51 determines that the contour C corresponding to the reference contour stored in the reference contour memory 54 is not detected by the contour detection unit 25, the notification unit 52 notifies the user to move the ultrasound probe 1 in order to acquire the ultrasound image in which the contour C corresponding to the reference contour is depicted. The notification unit 52 can notify the user, for example, by displaying a portion corresponding to the contour C that has not been detected, in a color different from the surrounding color on the image representing the three-dimensional shape of the contour C based on the three-dimensional image data of the contour C generated by the contour data generation unit 26.

The user can reliably perform the comprehensive scanning of the organ Q by reliably scanning the non-depicted portion by checking the notification to move the ultrasound probe 1 by the notification unit 52.

### Fifth Embodiment

In order for the user to clearly ascertain the contour C and the region R1 of the organ Q already scanned, the ultrasound diagnostic apparatus can also display the contour C and the region R1 of the organ Q already scanned in an emphasized manner on the monitor 23 in a three-dimensional schematic diagram that imitates the structure in the subject.

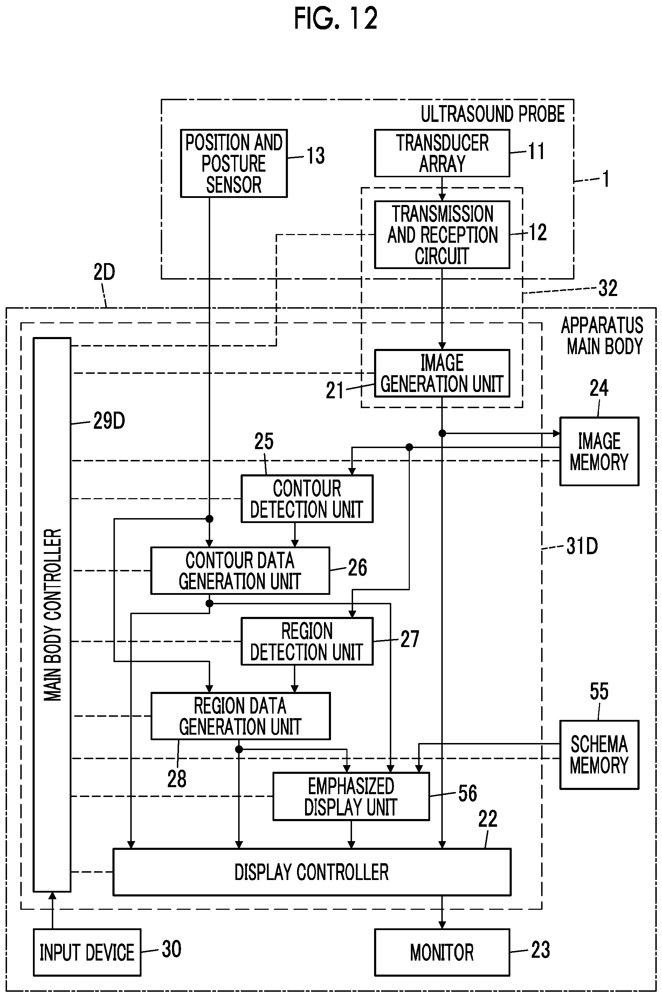

Fig. 12 is a block diagram illustrating a configuration of an ultrasound diagnostic apparatus according to a fifth embodiment. The ultrasound diagnostic apparatus of the fifth embodiment is obtained by including an apparatus main body 2D instead of the apparatus main body 2 in the ultrasound diagnostic apparatus of the first embodiment illustrated in Fig. 1. The apparatus main body 2D is obtained by further providing a schema memory 55 and an emphasized display unit 56 to the apparatus main body 2 in the first embodiment, and including a main body controller 29D instead of the main body controller 29.

In the apparatus main body 2D, the schema memory 55 is connected to the main body controller 29D. In addition, the emphasized display unit 56 is connected to the schema memory 55, the contour data generation unit 26, and the region data generation unit 28. The emphasized display unit 56 is connected to the display controller 22 and the main body controller 29D. In addition, the image generation unit 21, the display controller 22, the contour detection unit 25, the contour data generation unit 26, the region detection unit 27, the region data generation unit 28, the main body controller 29D, and the emphasized display unit 56 constitute a processor 31D for the apparatus main body 2D.

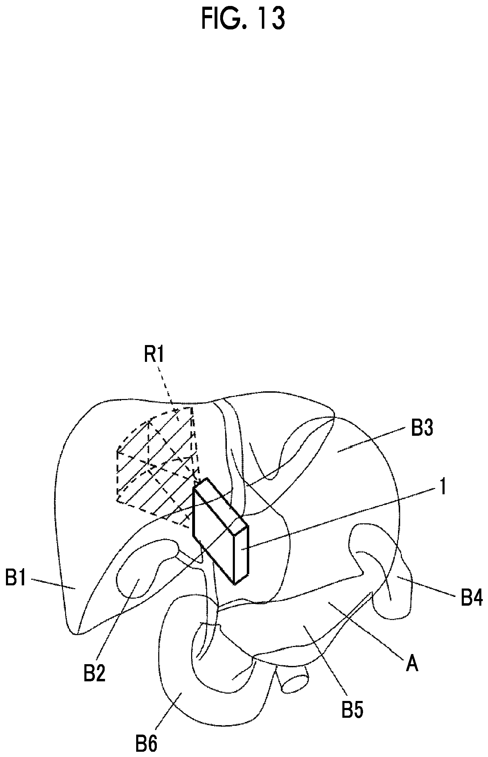

As illustrated in Fig. 13, for example, the schema memory 55 is a memory that stores a plurality of three-dimensional schema images A, which are three-dimensional schematic diagrams that imitate the structure including at least one organ Q in the subject, in advance. Here, Fig. 13 illustrates an example of the three-dimensional schema image A including a liver B1, a gallbladder B2, a stomach B3, a spleen B4, a pancreas B5, and a duodenum B6, as the organ Q. As the schema memory 55, for example, recording media such as a flash memory, an HDD, an SSD, an FD, an MO disk, an MT, a RAM, a CD, a DVD, an SD card, or a USB memory can be used.

As illustrated in Fig. 13, the emphasized display unit 56 displays a scanned portion P1 formed by the contour C detected by the contour detection unit 25 and the region R1 detected by the region detection unit 27 in the three-dimensional schema image A in an emphasized manner on the three-dimensional schema image A on the basis of the three-dimensional image data of the contour C and the region R1 of the organ Q generated by the contour data generation unit 26 and the region data generation unit 28. In the example of Fig. 13, the schematic diagram of the ultrasound probe 1 is illustrated to indicate that the scanned portion P1 corresponds to the ultrasound images of the plurality of frames generated while the ultrasound probe 1 is inclined in a certain angle range, but the schematic diagram of the ultrasound probe 1 may not be illustrated.

The emphasized display unit 56 can read out and display, for example, the three-dimensional schema image A designated by the user via the input device 30 among the plurality of three-dimensional schema images A stored in the schema memory 55, on the monitor 23. In addition, the emphasized display unit 56 can display the already scanned contour C and region R1 in an emphasized manner by giving a color different from the surrounding color to the scanned portion P1, blinking, or the like.

The user can clearly and easily ascertain a location of the subject at which the ultrasound image has already been captured by checking the scanned portion P1 displayed in an emphasized manner on the monitor 23. Accordingly, the user can reliably perform the comprehensive scanning of necessary examination locations.

Note that the example has been described in which the three-dimensional schema image A designated by the user via the input device 30 is read out from the plurality of three-dimensional schema images A stored in the schema memory 55, but, for example, the emphasized display unit 56 can automatically select the three-dimensional schema image A including the position of the scanned portion P1, from the schema memory 55 and can display the three-dimensional schema image A on the monitor 23.

### Sixth Embodiment

In the first to fifth embodiments, the description has been made in which the image representing the three-dimensional shapes of the contour C and the region R1 of the organ Q on the monitor 23, but the ultrasound diagnostic apparatus may display two-dimensional tomographic images of the contour C and the region R1 of the organ Q on the monitor 23.

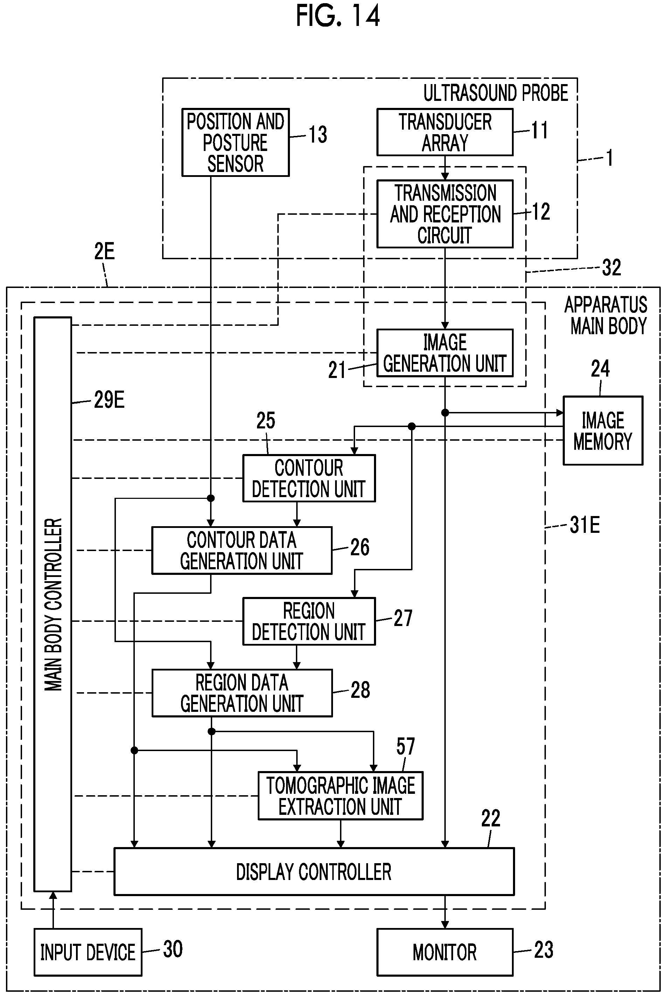

Fig. 14 is a block diagram illustrating a configuration of an ultrasound diagnostic apparatus according to a sixth embodiment. The ultrasound diagnostic apparatus of the sixth embodiment is obtained by including an apparatus main body 2E instead of the apparatus main body 2 in the ultrasound diagnostic apparatus of the first embodiment illustrated in Fig. 1. The apparatus main body 2E is obtained by further providing a tomographic image extraction unit 57 to the apparatus main body 2 in the first embodiment, and including a main body controller 29E instead of the main body controller 29.

In the apparatus main body 2E, the tomographic image extraction unit 57 is connected to the contour data generation unit 26 and the region data generation unit 28. The tomographic image extraction unit 57 is connected to the display controller 22 and the main body controller 29E. In addition, the image generation unit 21, the display controller 22, the contour detection unit 25, the contour data generation unit 26, the region detection unit 27, the region data generation unit 28, the main body controller 29E, and the tomographic image extraction unit 57 constitute a processor 31E for the apparatus main body 2E.

The tomographic image extraction unit 57 extracts a two-dimensional tomographic image of the contour C and a two-dimensional tomographic image of the region R1 on the same cut section, from the three-dimensional image data of the contour C generated by the contour data generation unit 26 and the three-dimensional image data of the region R1 generated by the region data generation unit 28, respectively. The tomographic image extraction unit 57 can extract, for example, three two-dimensional tomographic images of the contour C and the region R1 along the planes perpendicular to each other in the three-dimensional space, respectively.

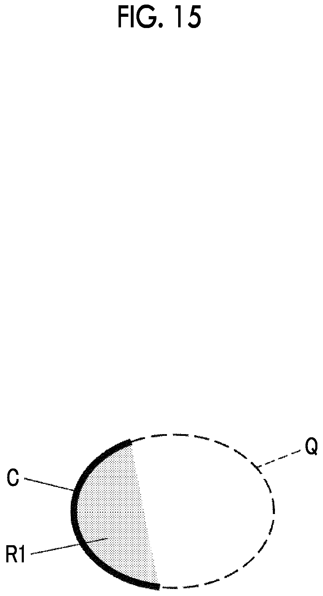

The display controller 22 sequentially displays, for example, the two-dimensional tomographic image of the contour C and the two-dimensional tomographic image of the region R1 extracted by the tomographic image extraction unit 57, as the image representing the contour C of the organ Q and the image representing the region R1, on the monitor 23 as illustrated in Fig. 15.

The user can reliably perform the comprehensive scanning of the organ Q while clearly ascertaining the scanned portion of the organ Q, by checking the two-dimensional tomographic images of the contour C and the region R1 of the organ Q displayed on the monitor 23.

Note that the tomographic image extraction unit 57 can change, for example, the position and the inclined angle of the cut section to be extracted in the organ Q on the basis of the input operation of the user via the input device 30. In this case, the display controller 22 can sequentially display the two-dimensional tomographic image of the contour C and the two-dimensional tomographic image of the region R1 in the cut section designated by the user via the input device 30, on the monitor 23. Accordingly, the user can more specifically ascertain a portion that has been scanned and a portion that has not been scanned in the organ Q.

### Explanation of References

1: ultrasound probe
2, 2A, 2B, 2C, 2D, 2E: apparatus main body
11: transducer array
12: transmission and reception circuit
13: position and posture sensor
21: image generation unit
22: display controller
23: monitor
24: image memory
25: contour detection unit
26: contour data generation unit
27: region detection unit
28: region data generation unit
29, 29A, 29B, 29C, 29D, 29E: main body controller
30: input device
31, 31A, 31B, 31C, 31D, 31E: processor
32: image acquisition unit
41: pulser
42: amplification unit
43: AD conversion unit
44: beam former
45: signal processing unit
46: DSC
47: image processing unit
51: non-depiction determination unit
52: notification unit
53: noncontact detection unit
54: reference contour memory
55: schema memory
56: emphasized display unit
57: tomographic image extraction unit
A: three-dimensional schema image
B1: liver
B2: gallbladder
B3: stomach
B4: spleen
B5: pancreas
B6: duodenum
D, D1: cell
D2: adjacent cell
G: three-dimensional grid
P1: scanned portion

## Claims

1. An ultrasound diagnostic apparatus comprising:
an ultrasound probe (1);
a position and posture sensor (13) that acquires position and posture information of the ultrasound probe (1);
an image acquisition unit (32) that acquires an ultrasound image of a subject by transmitting and receiving ultrasound beams by using the ultrasound probe (1);
a contour detection unit (25) that detects a contour of an organ imaged in the ultrasound image, on the basis of the ultrasound image;
a region detection unit (27) that detects a region of the organ imaged in the ultrasound image, on the basis of the ultrasound image;
a contour data generation unit (26) that generates three-dimensional image data of the contour of the organ on the basis of the contour detected by the contour detection unit (25) and the position and posture information acquired by the position and posture sensor (13);
a region data generation unit (28) that generates three-dimensional image data of the region of the organ on the basis of the region detected by the region detection unit (27) and the position and posture information acquired by the position and posture sensor (13); and
a monitor (23) that sequentially displays an image representing the contour and an image representing the region on the basis of the three-dimensional image data of the contour and the three-dimensional image data of the region; wherein
the contour detection unit (25) detects the contour by using a contour detection model in which the contour of the organ in the ultrasound image in which the organ is imaged is trained by learning a relationship between a large number of ultrasound images in which the organ is imaged and a contour of the organ in the ultrasound images in advance, and wherein the contour detection model outputs the contour of the organ in the ultrasound image in response to the input of the ultrasound image in which the organ is imaged, and
the region detection unit (27) detects the region by using a region detection model in which the region of the organ in the ultrasound image in which the organ is imaged is trained by learning a relationship between a large number of ultrasound images in which an organ is imaged and a region of the organ in the ultrasound images in advance, and wherein the region detection model outputs the region of the organ in the ultrasound image for each pixel in response to the input of the ultrasound image in which the organ is imaged.

2. The ultrasound diagnostic apparatus according to claim 1, further comprising:
a non-depiction determination unit (51) that determines a portion of which the contour is not detected by the contour detection unit (25) or a portion of which the region is not detected by the region detection unit (27), as a non-depicted portion; and
a notification unit (52) that notifies a user in a case where the non-depiction determination unit (51) determines that the non-depicted portion is present.

3. The ultrasound diagnostic apparatus according to claim 2,
wherein in a case where the contour detected by the contour detection unit (52) or the region detected by the region detection unit (27) has a discontinuous portion having an interval equal to or greater than a predetermined interval, the non-depiction determination unit (51) determines the discontinuous portion as the non-depicted portion.

4. The ultrasound diagnostic apparatus according to claim 2, further comprising:
a noncontact detection unit (53) that detects a noncontact state in which the ultrasound probe (1) is separated from a body surface of the subject,
wherein the non-depiction determination unit (51) determines that the non-depicted portion is present in a case where the noncontact state is detected by the noncontact detection unit (53).

5. The ultrasound diagnostic apparatus according to claim 2,
wherein the non-depiction determination unit (51) determines that the non-depicted portion is present in a case where a predetermined period of time has elapsed from a start of scanning by the ultrasound probe (1).

6. The ultrasound diagnostic apparatus according to claim 2,
wherein the non-depiction determination unit (51) determines that the non-depicted portion is present in a case where the user gives an instruction to complete an examination.

7. The ultrasound diagnostic apparatus according to claim 2,
wherein the non-depiction determination unit (51) determines that the non-depicted portion of which the region is not detected by the region detection unit (27) is present in a case where the contour detected by the contour detection unit (25) is closed.

8. The ultrasound diagnostic apparatus according to any one of claims 2 to 7,
wherein in a case where the contour detected by the contour detection unit (25) is closed and the region that is not detected by the region detection unit (27) is not present in a range inside the contour, the notification unit (52) notifies the user that scanning is completed.

9. The ultrasound diagnostic apparatus according to any one of claims 1 to 8,
wherein the monitor (23) displays a three-dimensional schema image, and
the ultrasound diagnostic apparatus further comprises an emphasized display unit (56) that displays the contour detected by the contour detection unit (25) and the region detected by the region detection unit (27) in an emphasized manner on the three-dimensional schema image.

10. The ultrasound diagnostic apparatus according to any one of claims 2 to 7, further comprising:
a reference contour memory (54) that stores a reference contour that is able to be depicted,
wherein in a case where a contour corresponding to the reference contour stored in the reference contour memory (54) is not detected by the contour detection unit (25), the notification unit (52) notifies the user to move the ultrasound probe (1).

11. The ultrasound diagnostic apparatus according to any one of claims 1 to 10,
wherein the monitor (23) sequentially displays a three-dimensional image of the contour and a three-dimensional image of the region, as the image representing the contour and the image representing the region.

12. The ultrasound diagnostic apparatus according to any one of claims 1 to 11, further comprising:
a tomographic image extraction unit (57) that extracts a two-dimensional tomographic image of the contour and a two-dimensional tomographic image of the region on a same cut section, from the three-dimensional image data of the contour generated by the contour data generation unit (26) and the three-dimensional image data of the region generated by the region data generation unit (28), respectively,
wherein the monitor (23) sequentially displays the two-dimensional tomographic image of the contour and the two-dimensional tomographic image of the region extracted by the tomographic image extraction unit (57), as the image representing the contour and the image representing the region.

13. The ultrasound diagnostic apparatus according to any one of claims 1 to 12,
wherein the monitor (23) displays the image representing the contour and the image representing the region in a superimposed manner.

14. The ultrasound diagnostic apparatus according to any one of claims 1 to 12,
wherein the monitor (23) displays the image representing the contour and the image representing the region side by side.

15. The ultrasound diagnostic apparatus according to any one of claims 1 to 14,
wherein the position and posture sensor includes an inertial sensor, a magnetic sensor, or an optical sensor.

16. A control method of an ultrasound diagnostic apparatus, the control method comprising:
acquiring position and posture information of an ultrasound probe (1);
acquiring an ultrasound image of a subject by transmitting and receiving ultrasound beams by using the ultrasound probe (1);
detecting a contour of an organ imaged in the ultrasound image, on the basis of the ultrasound image;
detecting a region of the organ imaged in the ultrasound image, on the basis of the ultrasound image;
generating three-dimensional image data of the contour of the organ on the basis of the detected contour and the acquired position and posture information;
generating three-dimensional image data of the region of the organ on the basis of the detected region and the acquired position and posture information; and
sequentially displaying an image representing the contour and an image representing the region on a monitor (23) on the basis of the three-dimensional image data of the contour and the three-dimensional image data of the region; wherein
detecting the contour of an organ imaged in the ultrasound image includes using a contour detection model in which the contour of the organ in the ultrasound image in which the organ is imaged is trained by learning a relationship between a large number of ultrasound images in which the organ is imaged and a contour of the organ in the ultrasound images in advance, and wherein the contour detection model outputs the contour of the organ in the ultrasound image in response to the input of the ultrasound image in which the organ is imaged; and
detecting the region of the organ imaged in the ultrasound image includes using a region detection model in which the region of the organ in the ultrasound image in which the organ is imaged is trained by learning a relationship between a large number of ultrasound images in which an organ is imaged and a region of the organ in the ultrasound images in advance, and wherein the region detection model outputs the region of the organ in the ultrasound image for each pixel in response to the input of the ultrasound image in which the organ is imaged.

## Patentansprüche

1. Ultraschalldiagnosevorrichtung, umfassend:
eine Ultraschallsonde (1);
einen Positions- und Ausrichtungssensor (13), der Positions- und
Ausrichtungsinformationen der Ultraschallsonde (1) erfasst;
eine Bilderfassungseinheit (32), die ein Ultraschallbild einer Untersuchungsperson durch Übertragen und Empfangen von Ultraschallstrahlen unter Verwendung der Ultraschallsonde (1) erfasst;
eine Konturdetektionseinheit (25), die eine Kontur eines Organs, das in dem Ultraschallbild abgebildet ist, auf der Grundlage des Ultraschallbildes detektiert;
eine Bereichsdetektionseinheit (27), die einen Bereich des Organs, das in dem Ultraschallbild abgebildet ist, auf der Grundlage des Ultraschallbildes detektiert;
eine Konturdaten-Erzeugungseinheit (26), die dreidimensionale Bilddaten der Kontur des Organs auf der Grundlage der von der Konturdetektionseinheit (25) detektierten Kontur und der von dem Positions- und Ausrichtungssensor (13) erfassten Positions- und Ausrichtungsinformationen erzeugt;
eine Bereichsdaten-Erzeugungseinheit (28), die dreidimensionale Bilddaten des Bereichs des Organs auf der Grundlage des von der Bereichsdetektionseinheit (27) detektierten Bereichs und der von dem Positions- und Ausrichtungssensor (13) erfassten Positions- und Ausrichtungsinformationen erzeugt; und
einen Monitor (23), der ein Bild, das die Kontur darstellt, und ein Bild, das den Bereich darstellt, auf der Grundlage der dreidimensionalen Bilddaten der Kontur und der dreidimensionalen Bilddaten des Bereichs nacheinander anzeigt; wobei
die Konturdetektionseinheit (25) die Kontur durch Verwenden eines Konturdetektionsmodells detektiert, bei dem die Kontur des Organs in dem Ultraschallbild, in dem das Organ abgebildet ist, im Voraus durch Lernen einer Beziehung zwischen einer großen Anzahl von Ultraschallbildern, in denen das Organ abgebildet ist, und einer Kontur des Organs in den Ultraschallbildern trainiert wird, und wobei das Konturdetektionsmodell die Kontur des Organs in dem Ultraschallbild als Reaktion auf die Eingabe des Ultraschallbildes, in dem das Organ abgebildet ist, ausgibt, und
die Bereichsdetektionseinheit (27) den Bereich durch Verwenden eines Bereichsdetektionsmodells detektiert, bei dem der Bereich des Organs in dem Ultraschallbild, in dem das Organ abgebildet ist, im Voraus durch Lernen einer Beziehung zwischen einer großen Anzahl von Ultraschallbildern, in denen ein Organ abgebildet ist, und einem Bereich des Organs in den Ultraschallbildern trainiert wird, und wobei das Bereichsdetektionsmodell den Bereich des Organs in dem Ultraschallbild für jedes Pixel als Reaktion auf die Eingabe des Ultraschallbildes, in dem das Organ abgebildet ist, ausgibt.

2. Ultraschalldiagnosevorrichtung nach Anspruch 1, ferner umfassend:
eine Nichtdarstellungsbestimmungseinheit (51), die einen Abschnitt, von dem die Kontur nicht von der Konturdetektionseinheit (25) detektiert wird, oder einen Abschnitt, von dem der Bereich nicht von der Bereichsdetektionseinheit (27) detektiert wird, als einen nicht dargestellten Abschnitt bestimmt; und
eine Benachrichtigungseinheit (52), die einen Benutzer in einem Fall benachrichtigt, in dem die Nichtdarstellungsbestimmungseinheit (51) bestimmt, dass der nicht dargestellte Abschnitt vorhanden ist.

3. Ultraschalldiagnosevorrichtung nach Anspruch 2,
wobei in einem Fall, in dem die von der Konturdetektionseinheit (52) detektierte Kontur oder der von der Bereichsdetektionseinheit (27) detektierte Bereich einen diskontinuierlichen Abschnitt mit einem Abstand, der gleich oder größer als ein vorbestimmter Abstand ist, aufweist, die Nichtdarstellungsbestimmungseinheit (51) den diskontinuierlichen Abschnitt als den nicht dargestellten Abschnitt bestimmt.

4. Ultraschalldiagnosevorrichtung nach Anspruch 2, ferner umfassend:
eine Nichtkontaktdetektionseinheit (53), die einen Nichtkontaktzustand detektiert, in dem die Ultraschallsonde (1) von einer Körperoberfläche der Untersuchungsperson getrennt ist,
wobei die Nichtdarstellungsbestimmungseinheit (51) bestimmt, dass der nicht dargestellte Abschnitt in einem Fall vorhanden ist, in dem der Nichtkontaktzustand von der Nichtkontaktdetektionseinheit (53) detektiert wird.

5. Ultraschalldiagnosevorrichtung nach Anspruch 2,
wobei die Nichtdarstellungsbestimmungseinheit (51) bestimmt, dass der nicht dargestellte Abschnitt in einem Fall vorhanden ist, in dem eine vorbestimmte Zeitspanne seit einem Start des Abtastens durch die Ultraschallsonde (1) verstrichen ist.

6. Ultraschalldiagnosevorrichtung nach Anspruch 2,
wobei die Nichtdarstellungsbestimmungseinheit (51) bestimmt, dass der nicht dargestellte Abschnitt in einem Fall vorhanden ist, in dem der Benutzer eine Anweisung zum Abschließen einer Untersuchung gibt.

7. Ultraschalldiagnosevorrichtung nach Anspruch 2,
wobei die Nichtdarstellungsbestimmungseinheit (51) bestimmt, dass der nicht dargestellte Abschnitt, von dem der Bereich nicht von der Bereichsdetektionseinheit (27) detektiert wird, in einem Fall vorhanden ist, in dem die von der Konturdetektionseinheit (25) detektierte Kontur geschlossen ist.

8. Ultraschalldiagnosevorrichtung nach einem der Ansprüche 2 bis 7,
wobei in einem Fall, in dem die von der Konturdetektionseinheit (25) detektierte Kontur geschlossen ist und der Bereich, der nicht von der Bereichsdetektionseinheit (27) detektiert wird, nicht in einem Bereich innerhalb der Kontur vorhanden ist, die Benachrichtigungseinheit (52) den Benutzer darüber benachrichtigt, dass Abtasten abgeschlossen ist.

9. Ultraschalldiagnosevorrichtung nach einem der Ansprüche 1 bis 8,
wobei der Monitor (23) ein dreidimensionales Schemabild anzeigt, und
die Ultraschalldiagnosevorrichtung ferner eine hervorgehobene Anzeigeeinheit (56) umfasst, die die von der Konturdetektionseinheit (25) detektierte Kontur und den von der Bereichsdetektionseinheit (27) detektierten Bereich auf dem dreidimensionalen Schemabild hervorgehoben anzeigt.

10. Ultraschalldiagnosevorrichtung nach einem der Ansprüche 2 bis 7, ferner umfassend:
einen Referenzkontur-Speicher (54), der eine Referenzkontur speichert, die dargestellt werden kann,
wobei in einem Fall, in dem eine Kontur, die der in dem Referenzkontur-Speicher (54) gespeicherten Referenzkontur entspricht, von der Konturdetektionseinheit (25) nicht detektiert wird, die Benachrichtigungseinheit (52) den Benutzer darüber benachrichtigt, die Ultraschallsonde (1) zu bewegen.

11. Ultraschalldiagnosevorrichtung nach einem der Ansprüche 1 bis 10,
wobei der Monitor (23) ein dreidimensionales Bild der Kontur und ein dreidimensionales Bild des Bereichs als das Bild, das die Kontur darstellt, und das Bild, das den Bereich darstellt, nacheinander anzeigt.

12. Ultraschalldiagnosevorrichtung nach einem der Ansprüche 1 bis 11, ferner umfassend:
eine Tomographiebild-Extraktionseinheit (57), die ein zweidimensionales Tomographiebild der Kontur und ein zweidimensionales Tomographiebild des Bereichs auf einem gleichen Schnittabschnitt jeweils aus den dreidimensionalen Bilddaten der Kontur, die von der Konturdaten-Erzeugungseinheit (26) erzeugt worden sind, und den dreidimensionalen Bilddaten des Bereichs, die von der Bereichsdaten-Erzeugungseinheit (28) erzeugt worden sind, extrahiert,
wobei der Monitor (23) das zweidimensionale Tomographiebild der Kontur und das zweidimensionale Tomographiebild des Bereichs, die von der Tomographiebild-Extraktionseinheit (57) extrahiert worden sind, als das Bild, das die Kontur darstellt, und das Bild, das den Bereich darstellt, nacheinander anzeigt,

13. Ultraschalldiagnosevorrichtung nach einem der Ansprüche 1 bis 12,
wobei der Monitor (23) das Bild, das die Kontur darstellt, und das Bild, das den Bereich darstellt, überlagert anzeigt.

14. Ultraschalldiagnosevorrichtung nach einem der Ansprüche 1 bis 12,
wobei der Monitor (23) das Bild, das die Kontur darstellt, und das Bild, das den Bereich darstellt, Seite an Seite anzeigt.

15. Ultraschalldiagnosevorrichtung nach einem der Ansprüche 1 bis 14,
wobei der Positions- und Ausrichtungssensor einen Trägheitssensor, einen Magnetsensor oder einen optischen Sensor enthält.

16. Steuerverfahren einer Ultraschalldiagnosevorrichtung, wobei das Steuerverfahren umfasst:
Erfassen von Positions- und Ausrichtungsinformationen einer Ultraschallsonde (1);
Erfassen eines Ultraschallbildes einer Untersuchungsperson durch Übertragen und
Empfangen von Ultraschallstrahlen durch Verwenden der Ultraschallsonde (1);
Detektieren einer Kontur eines Organs, das in dem Ultraschallbild abgebildet ist, auf der Grundlage des Ultraschallbildes;
Detektieren eines Bereichs des Organs, das in dem Ultraschallbild abgebildet ist, auf der Grundlage des Ultraschallbildes;
Erzeugen von dreidimensionalen Bilddaten der Kontur des Organs auf der Grundlage der detektierten Kontur und der erfassten Positions- und Ausrichtungsinformationen;
Erzeugen von dreidimensionalen Bilddaten des Bereichs des Organs auf der Grundlage des detektierten Bereichs und der erfassten Positions- und Ausrichtungsinformationen; und
Anzeigen eines Bildes, das die Kontur darstellt, und eines Bildes, das den Bereich darstellt, auf einem Monitor (23) nacheinander auf der Grundlage der dreidimensionalen Bilddaten der Kontur und der dreidimensionalen Bilddaten des Bereichs; wobei
Detektieren der Kontur eines Organs, das in dem Ultraschallbild abgebildet ist, enthält Verwenden eines Konturdetektionsmodells, bei dem die Kontur des Organs in dem Ultraschallbild, in dem das Organ abgebildet ist, durch Lernen einer Beziehung zwischen einer großen Anzahl von Ultraschallbildern, in denen das Organ abgebildet ist, und einer Kontur des Organs in den Ultraschallbildern im Voraus trainiert wird, und wobei das Konturdetektionsmodell die Kontur des Organs in dem Ultraschallbild als Reaktion auf die Eingabe des Ultraschallbildes, in dem das Organ abgebildet ist, ausgibt; und
Detektieren des Bereichs des Organs, das in dem Ultraschallbild abgebildet ist, enthält Verwenden eines Bereichsdetektionsmodells, bei dem der Bereich des Organs in dem Ultraschallbild, in dem das Organ abgebildet ist, durch Lernen einer Beziehung zwischen einer großen Anzahl von Ultraschallbildern, in denen ein Organ abgebildet ist, und einem Bereich des Organs in den Ultraschallbildern im Voraus trainiert wird, und wobei das Bereichsdetektionsmodell den Bereich des Organs in dem Ultraschallbild für jedes Pixel als Reaktion auf die Eingabe des Ultraschallbildes, in dem das Organ abgebildet ist, ausgibt.

## Revendications

1. Appareil de diagnostic par ultrasons comprenant :
une sonde ultrasonore (1) ;
un capteur de position et de posture (13) qui acquiert des informations de position et de posture de la sonde ultrasonore (1) ;
une unité d'acquisition d'images (32) qui acquiert une image ultrasonore d'un sujet en transmettant et en recevant des faisceaux ultrasonores à l'aide de la sonde ultrasonore (1) ;
une unité de détection de contour (25) qui détecte un contour d'un organe imagé dans l'image ultrasonore, sur la base de l'image ultrasonore ;
une unité de détection de région (27) qui détecte une région de l'organe imagé dans l'image ultrasonore, sur la base de l'image ultrasonore ;
une unité de génération de données de contour (26) qui génère des données d'images tridimensionnelles du contour de l'organe sur la base du contour détecté par l'unité de détection de contour (25) et des informations de position et de posture acquises par le capteur de position et de posture (13) ;
une unité de génération de données de région (28) qui génère des données d'images tridimensionnelles de la région de l'organe sur la base de la région détectée par l'unité de détection de région (27) et des informations de position et de posture acquises par le capteur de position et de posture (13) ; et
un moniteur (23) qui affiche séquentiellement une image représentant le contour et une image représentant la région sur la base des données d'images tridimensionnelles du contour et des données d'images tridimensionnelles de la région ; dans lequel
l'unité de détection de contour (25) détecte le contour à l'aide d'un modèle de détection de contour dans lequel le contour de l'organe dans l'image ultrasonore où l'organe est imagé est entraîné en apprenant à l'avance une relation entre un grand nombre d'images ultrasonores où l'organe est imagé et un contour de l'organe dans les images ultrasonores, et dans lequel le modèle de détection de contour produit le contour de l'organe dans l'image ultrasonore en réponse à l'entrée de l'image ultrasonore où l'organe est imagé, et
l'unité de détection de région (27) détecte la région à l'aide d'un modèle de détection de région dans lequel la région de l'organe dans l'image ultrasonore où l'organe est imagé est entraînée en apprenant à l'avance une relation entre un grand nombre d'images ultrasonores où un organe est imagé et une région de l'organe dans les images ultrasonores, et dans lequel le modèle de détection de région produit la région de l'organe dans l'image ultrasonore pour chaque pixel en réponse à l'entrée de l'image ultrasonore où l'organe est imagé.

2. Appareil de diagnostic par ultrasons selon la revendication 1, comprenant en outre :
une unité de détermination de non-représentation (51) qui détermine, comme portion non représentée, une portion dont le contour n'est pas détecté par l'unité de détection de contour (25) ou une portion dont la région n'est pas détectée par l'unité de détection de région (27) ; et
une unité de notification (52) qui notifie un utilisateur dans un cas où l'unité de détermination de non-représentation (51) détermine que la portion non représentée est présente.

3. Appareil de diagnostic par ultrasons selon la revendication 2,
dans lequel, dans un cas où le contour détecté par l'unité de détection de contour (52) ou la région détectée par l'unité de détection de région (27) comporte une portion discontinue ayant un intervalle égal ou supérieur à un intervalle prédéterminé, l'unité de détermination de non-représentation (51) détermine la portion discontinue comme la portion non représentée.

4. Appareil de diagnostic par ultrasons selon la revendication 2, comprenant en outre :
une unité de détection de non-contact (53) qui détecte un état de non-contact dans lequel la sonde ultrasonore (1) est séparée d'une surface corporelle du sujet,
dans lequel l'unité de détermination de non-représentation (51) détermine que la portion non représentée est présente dans un cas où l'état de non-contact est détecté par l'unité de détection de non-contact (53).

5. Appareil de diagnostic par ultrasons selon la revendication 2,
dans lequel l'unité de détermination de non-représentation (51) détermine que la portion non représentée est présente dans un cas où une période de temps prédéterminée s'est écoulée depuis un début de balayage par la sonde ultrasonore (1).

6. Appareil de diagnostic par ultrasons selon la revendication 2,
dans lequel l'unité de détermination de non-représentation (51) détermine que la portion non représentée est présente dans un cas où l'utilisateur donne une instruction de terminer un examen.

7. Appareil de diagnostic par ultrasons selon la revendication 2,
dans lequel l'unité de détermination de non-représentation (51) détermine que la portion non représentée dont la région n'est pas détectée par l'unité de détection de région (27) est présente dans un cas où le contour détecté par l'unité de détection de contour (25) est fermé.

8. Appareil de diagnostic par ultrasons selon l'une quelconque des revendications 2 à 7,
dans lequel, dans un cas où le contour détecté par l'unité de détection de contour (25) est fermé et où la région qui n'est pas détectée par l'unité de détection de région (27) n'est pas présente dans une plage à l'intérieur du contour, l'unité de notification (52) notifie à l'utilisateur que le balayage est terminé.

9. Appareil de diagnostic par ultrasons selon l'une quelconque des revendications 1 à 8,
dans lequel le moniteur (23) affiche une image schématique tridimensionnelle, et
l'appareil de diagnostic par ultrasons comprend en outre une unité d'affichage avec mise en évidence (56) qui affiche le contour détecté par l'unité de détection de contour (25) et la région détectée par l'unité de détection de région (27) de manière mise en évidence sur l'image schématique tridimensionnelle.

10. Appareil de diagnostic par ultrasons selon l'une quelconque des revendications 2 à 7, comprenant en outre :
une mémoire de contour de référence (54) qui stocke un contour de référence pouvant être représenté,
dans lequel, dans un cas où un contour correspondant au contour de référence stocké dans la mémoire de contour de référence (54) n'est pas détecté par l'unité de détection de contour (25), l'unité de notification (52) notifie à l'utilisateur de déplacer la sonde ultrasonore (1).

11. Appareil de diagnostic par ultrasons selon l'une quelconque des revendications 1 à 10, dans lequel le moniteur (23) affiche séquentiellement une image tridimensionnelle du contour et une image tridimensionnelle de la région comme l'image représentant le contour et l'image représentant la région.

12. Appareil de diagnostic par ultrasons selon l'une quelconque des revendications 1 à 11, comprenant en outre :
une unité d'extraction d'images tomographiques (57) qui extrait, respectivement, une image tomographique bidimensionnelle du contour et une image tomographique bidimensionnelle de la région sur une même section de coupe, à partir des données d'images tridimensionnelles du contour générées par l'unité de génération de données de contour (26) et des données d'images tridimensionnelles de la région générées par l'unité de génération de données de région (28),
dans lequel le moniteur (23) affiche séquentiellement l'image tomographique bidimensionnelle du contour et l'image tomographique bidimensionnelle de la région extraites par l'unité d'extraction d'images tomographiques (57), comme l'image représentant le contour et l'image représentant la région.

13. Appareil de diagnostic par ultrasons selon l'une quelconque des revendications 1 à 12,
dans lequel le moniteur (23) affiche l'image représentant le contour et l'image représentant la région de manière superposée.

14. Appareil de diagnostic par ultrasons selon l'une quelconque des revendications 1 à 12,
dans lequel le moniteur (23) affiche côte à côte l'image représentant le contour et l'image représentant la région.

15. Appareil de diagnostic par ultrasons selon l'une quelconque des revendications 1 à 14,
dans lequel le capteur de position et de posture inclut un capteur inertiel, un capteur magnétique ou un capteur optique.

16. Procédé de contrôle d'un appareil de diagnostic par ultrasons, le procédé de contrôle comprenant :
acquérir des informations de position et de posture d'une sonde ultrasonore (1) ;
acquérir une image ultrasonore d'un sujet en transmettant et en recevant des faisceaux ultrasonores à l'aide de la sonde ultrasonore (1) ;
détecter un contour d'un organe imagé dans l'image ultrasonore, sur la base de l'image ultrasonore ;
détecter une région de l'organe imagé dans l'image ultrasonore, sur la base de l'image ultrasonore ;
générer des données d'images tridimensionnelles du contour de l'organe sur la base du contour détecté et des informations de position et de posture acquises ;
générer des données d'images tridimensionnelles de la région de l'organe sur la base de la région détectée et des informations de position et de posture acquises ; et
afficher séquentiellement, sur un moniteur (23), une image représentant le contour et
une image représentant la région sur la base des données d'images tridimensionnelles du contour et des données d'images tridimensionnelles de la région ; dans lequel
détecter le contour d'un organe imagé dans l'image ultrasonore inclut l'utilisation d'un modèle de détection de contour dans lequel le contour de l'organe dans l'image ultrasonore où l'organe est imagé est entraîné en apprenant à l'avance une relation entre un grand nombre d'images ultrasonores où l'organe est imagé et un contour de l'organe dans les images ultrasonores, et dans lequel le modèle de détection de contour produit le contour de l'organe dans l'image ultrasonore en réponse à l'entrée de l'image ultrasonore où l'organe est imagé ; et
détecter la région de l'organe imagé dans l'image ultrasonore inclut l'utilisation d'un modèle de détection de région dans lequel la région de l'organe dans l'image ultrasonore où l'organe est imagé est entraînée en apprenant à l'avance une relation entre un grand nombre d'images ultrasonores où un organe est imagé et une région de l'organe dans les images ultrasonores, et dans lequel le modèle de détection de région produit la région de l'organe dans l'image ultrasonore pour chaque pixel en réponse à l'entrée de l'image ultrasonore où l'organe est imagé.
